(19) Europäisches Patentamt
European Patent Office
Office européen des brevets

(11) Veröffentlichungsnummer : **0 315 748 B1**

(12) # EUROPÄISCHE PATENTSCHRIFT

(45) Veröffentlichungstag der Patentschrift : **20.12.95 Patentblatt 95/51**

(51) Int. Cl.$^6$ : **G03F 7/004,** C07C 43/315, C07C 43/32, C07C 45/72, C07C 49/84

(21) Anmeldenummer : **88114590.8**

(22) Anmeldetag : **07.09.88**

(54) **Positiv arbeitendes strahlungsempfindliches Gemisch und daraus hergestelltes strahlungsempfindliches Aufzeichungsmaterial**

(30) Priorität : **13.09.87 DE 3730787**

(43) Veröffentlichungstag der Anmeldung : **17.05.89 Patentblatt 89/20**

(45) Bekanntmachung des Hinweises auf die Patenterteilung : **20.12.95 Patentblatt 95/51**

(84) Benannte Vertragsstaaten : **DE FR GB IT**

(56) Entgegenhaltungen :
**EP-A- 0 006 627**
**EP-A- 0 202 196**
**FR-A- 2 305 757**
**FR-A- 2 389 155**

(73) Patentinhaber : **HOECHST AKTIENGESELLSCHAFT**
**D-65926 Frankfurt am Main (DE)**

(72) Erfinder : **Dössel, Karl-Friedrich, Dr.**
**Fritz-Kalle-Strasse 8**
**D-6200 Wiesbaden (DE)**
Erfinder : **Dammel, Ralph, Dr.**
**Eibenweg 39**
**D-6500 Mainz-Bretzenheim (DE)**
Erfinder : **Lingnau, Jürgen, Dr.**
**Karolingerstrasse 10**
**D-6500 Mainz-Laubenheim (DE)**



Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue Saint-Denis, 75001 PARIS

## Beschreibung

Die Erfindung betrifft ein positiv arbeitendes strahlungsempfindliches Gemisch enthaltend eine Verbindung, die unter Einwirkung von aktinischer Strahlung eine Säure bildet, sowie eine Verbindung, die durch Säure spaltbar ist.

Positiv arbeitende strahlungsempfindliche Gemische, bei denen durch Einwirkung von aktinischer Strahlung auf einen Photoinitiator eine Säure gebildet wird und diese dann in einer Sekundärreaktion ein säurespaltbares Material in den bestrahlten Bereichen in einem entsprechenden Entwickler löslich macht, sind seit langem bekannt.

Unter den bisher auf diesem Gebiet eingesetzten säurespaltbaren Materialien haben sich bewährt:

a) solche mit mindestens einer Orthocarbonsäureester-und/oder Carbonsäureamidacetalgruppierung, wobei die Verbindungen auch polymeren Charakter haben und die genannten Gruppierungen als verknüpfende Elemente in der Hauptkette oder als seitenständige Substituenten auftreten können,

b) Oligomer- oder Polymerverbindungen mit wiederkehrenden Acetal- und/oder Ketalgruppierungen in der Hauptkette,

c) Verbindungen mit mindestens einer Enolether- oder N-Acyliminocarbonatgruppierung,

d) Verbindungen mit Silylethergruppierungen,

e) Verbindungen mit Silylenolethergruppierungen und

f) cyclische Acetale oder Ketale von β-Ketoestern oder -amiden.

Durch Säure spaltbare Verbindungen des Typs a) als Komponenten strahlungsempfindlicher Gemische sind in der EP-A-0 022 571 und der DE-A-26 10 842 ausführlich beschrieben; Gemische, die Verbindungen des Typs b) enthalten, sind in der DE-C-23 06 248 und der DE-C-27 18 254 beschrieben; Verbindungen des Typs c) werden in den EP-A-0 006 626 und 0 006 627 genannt; Verbindungen, die dem Typ d) zuzurechnen sind, werden in den DE-A-35 44 165 und DE-A-36 01 264 vorgestellt, Verbindungen des Typs e) findet man in der gleichzeitig eingereichten Patentanmeldung DE-A-37 30 783; Verbindungen vom Typ f), sind in der EP-A-0 202 196 erwähnt.

In der DE-A-26 10 842 werden Verbindungen mit Orthoester- oder -amidacetalgruppierungen in einer strahlungsempfindlichen Kopiermasse beschrieben, die ihre Anwendung sowohl in der Herstellung von Druckplatten als auch von mikroelektronischen Schaltungen finden. Als Alkoholkomponenten derartiger Verbindungen werden Alkyl-, Alkenyl- und Arylreste genannt. Gute Resultate werden im Bereich aliphatischer Alkohole für $C_1$ bis $C_{18}$-Alkohole berichtet. Vergleichbare Eigenschaften sollen auch den Verbindungen auf der Basis von Monoalkylethern der Polyethylenglykole zukommen, wobei die Zahl der Ethylenoxydeinheiten insbesondere zwischen 1 und 8 liegt. Untersuchungen haben nun gezeigt, daß gute Verarbeitungstoleranzen immer dann erreicht werden, wenn die eingesetzte Alkoholkomponente einen Siedepunkt aufweist, der hinreichend hoch ist, um ein Verdunsten aus der bestrahlten Schicht zu verhindern. Dies führt bei Verwendung von Polyethylenglykolmonoalkylethern wie bei Verwendung von aliphatischen Alkoholen dazu, daß relativ hochmolekulare Alkoholkomponenten eingesetzt werden müssen. Diese wiederum ergeben Schwierigkeiten hinsichtlich der Verträglichkeit mit den in derartigen Schichten üblicherweise eingesetzten Phenolharzen bzw. durch ihre Wirksamkeit als Weichmacher für die Harzmatrix. Schließlich wird durch langkettige aliphatische Alkohole die Ätzresistenz von Schichten herabgesetzt. Solche Schichten überstehen daher einzelne Prozeßschritte bei der Herstellung z. B. mikroelektronischer Bauelemente nur unter Auflösungsverlust.

Auch die in der DE-A-27 18 254 beschriebenen strahlungsempfindlichen Kopiermassen sollen für lithographische Druckplatten wie auch für Positivresists eingesetzt werden. Sie enthalten als säurespaltbare Komponente eine (oligomere) Verbindung mit wiederkehrenden Acetal- oder Ketaleinheiten, deren Alkoholkomponente mindestens zweiwertig sein muß. Neben der durch den Herstellungsprozeß bedingten großen Schwankungsbreite der Molekulargewichte bzw. Polymerisationsgrade derartiger Polyacetale ist bei diesen säurespaltbaren Verbindungen die Gefahr des Eintrags von Verunreinigungen in das strahlungsempfindliche Gemisch besonders kritisch, weil eine destillative Reinigung nicht mehr möglich ist. Durch den höheren Polymerisationsgrad besteht bei den hier aufgeführten polymeren Acetalen und Ketalen zudem - je nach Applikationsbedingungen - eine erhöhte Gefahr der Entmischung mit dem eingesetzten Polymeren. Dies führt insbesondere bei der Anwendung im Sub-1µm-Bereich zu Auflösungsverlusten.

Daneben wird in neuerer Zeit - abweichend vom bisherigen Stand der Technik - vorgeschlagen, cyclische Acetale oder Ketale von β-Ketoestern oder -amiden als säurespaltbares Material, das auch für die Anwendung in Photoresists geeignet sein soll, einzusetzen (EP-A-0 202 196). Ein Nachteil dieser Photoresists ist ihre geringe Strahlungsempfindlichkeit.

Es bestand daher die Aufgabe, ein positiv arbeitendes strahlungsempfindliches Gemisch bereitzustellen, das sich in möglichst reiner, einheitlicher Form herstellen läßt, das während unterschiedlicher "Haltezeiten" zwischen Bestrahlung und Entwicklung keine Änderungen hinsichtlich der Entwicklungszeit aufzeigt, d.h. einen großen Verarbeitungsspielraum aufweist, die Anforderungen an eine hohe Strukturauflösung des entwickelten Resists erfüllt und diese auch während der folgenden Prozeßschritte behält, sowie eine einheitlich gute Entwickelbarkeit sowohl in metallionenhaltigen als auch in metallionenfreien, wäßrig-alkalischen Entwicklern aufweist.

Gelöst wird diese Aufgabe durch das Bereitstellen eines positiv arbeitenden strahlungsempfindlichen Gemisches, das eine Verbindung enthält, die unter Einwirkung von aktinischer Strahlung eine Säure bildet, und eine Verbindung, die durch Säure spaltbar ist, dadurch gekennzeichnet, daß die durch Säure spaltbare Verbindung ein Acetal oder Ketal ist, das aus einem Aldehyd oder einem Keton und einem Alkohol der allgemeinen Formel I

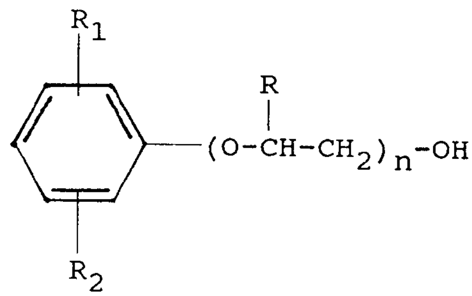
(I)

gebildet wird, in der

R Wasserstoff oder Alkyl darstellt,

$R_1$ und $R_2$ gleich oder verschieden sind und Wasserstoff, Hydroxy, Halogen, Cyano, Nitro, Alkyl, Alkoxy, Aryl, substituiertes Carbonyl, insbesondere Aryl- oder Alkylcarbonyl bedeuten, oder zusammen einen Ring formen, der mit $R_1$ und/oder $R_2$ substituiert sein kann, und

n 1 bis 3 ist.

Besonders bevorzugt ist, wenn

R Wasserstoff oder Methyl,

$R_1$ und $R_2$ gleich sind und Wasserstoff bedeuten oder verschieden sind und ein Rest davon Wasserstoff, der andere aber $(C_6\text{-}C_{10})$Aryl, insbesondere Phenyl, ggf. verzweigtes $(C_1\text{-}C_8)$Alkyl, insbesondere $(C_1\text{-}C_4)$Alkyl, $(C_6\text{-}C_{10})$Arylcarbonyl, insbesondere Phenylcarbonyl oder $(C_1\text{-}C_8)$Alkylcarbonyl, Halogen, insbesondere Brom oder Chlor, ist, sowie

n 1 bis 3

bedeuten. Sind $R_1$ und $R_2$ verschieden und bedeutet ein Rest davon Wasserstoff, so befindet sich der andere Rest bevorzugt in para-Stellung, wobei

R Wasserstoff oder Methyl und

n 1

bedeuten. Sind $R_1$ und $R_2$ gleich und bedeuten insbesondere Wasserstoff, so sind

R bevorzugt Wasserstoff oder Methyl und

n 1 bis 3.

Ganz besonders bevorzugt sind Acetale bzw. Ketale, deren Alkoholkomponente einen Siedepunkt bei 760 Torr von größer als 200 °C aufweist oder bei Raumtemperatur einen Feststoff darstellt.

Besonders günstige Eigenschaften werden bei Verwendung der unsubstituierten Verbindung der allgemeinen Formel I, des Phenylglykols als Alkoholkomponente, erzielt, wobei n = 1 gilt.

Als durch Säure spaltbare Verbindungen kommen in dem erfindungsgemäßen strahlungsempfindlichen Gemisch sowohl Acetale und Ketale, als auch Orthocarbonsäureester und -amidacetale zur Anwendung. Bevorzugt sind solche spaltbaren Verbindungen, die die säurespaltbaren Molekülgruppierungen ein- oder zweimal, besonders bevorzugt solche, die eine säurespaltbare Molekülgruppierung pro Molekül enthalten. Bei diesen Ausgangsverbindungen ist eine effiziente Reinigung der Produkte, z. B. auf destillativem Wege, sichergestellt.

Bevorzugt sind Acetale und Ketale, wobei es sich als besonders günstig erweist, wenn die diesen Acetalen bzw. Ketalen zugrunde liegenden Carbonylverbindungen einen Siedepunkt von oberhalb 155 °C und eine Löslichkeit in einem wäßrig-alkalischen Entwickler zwischen 0,1 und 100 g/l, wie in der gleichzeitig eingereichten Patentanmeldung EP-A-0 312 751 beschrieben, im Zusammenwirken mit den hier beanspruchten Alkoholkomponenten aufweisen.

Der Gehalt der durch Säure spaltbaren Komponenten im erfindungsgemäßen strahlungsempfindlichen

Gemisch beträgt 2 bis 30 Gew.-%, vorzugsweise 10 bis 25 Gew.-%, jeweils bezogen auf das Gesamtgewicht der Schichten.

Als Komponenten, die im erfindungsgemäßen strahlungsempfindlichen Gemisch beim Bestrahlen vorzugsweise eine starke Säure bilden bzw. abspalten, kommen eine große Anzahl von Verbindungen und Mischungen derselben in Frage.

Zu nennen sind Diazonium-, Phosphonium-, Sulfonium- und Iodonium-Salze, Halogenverbindungen, o-Chinondiazidsulfochloride und -ester sowie Organometall-Organohalogen-Kombinationen.

Die genannten Diazonium-, Phosphonium-, Sulfonium- und Iodonium-Verbindungen werden in der Regel eingesetzt in Form ihrer in organischen Lösungsmitteln löslichen Salze, meist als Abscheidungsprodukt mit komplexen Säuren wie Borfluorwasserstoffsäure, Hexafluorphosphor-, Hexafluorantimon- und -arsensäure. U.a. werden diese Verbindungen auch in der DE-A-36 01 264 genannt.

Unter die Halogenverbindungen zählen insbesondere Triazinderivate, die aus den US-A-3 515 552, 3 536 489 und 3 779 778 sowie den DE-C-27 18 259, 33 37 024, 33 33 450, 23 06 248, 22 43 621 und 12 98 414 bekannt sind. Diese können aber auch in Kombination mit anderen Photoinitiatoren wie Oxazolen, Oxadiazolen oder Thiazolen, aber auch untereinander in Mischungen eingesetzt werden.

Es sind aber auch Oxazole, Oxadiazole, Thiazole oder 2-Pyrone bekannt, die Trichlor- oder Tribrommethylgruppen enthalten (DE-A-30 21 599, 30 21 590, 28 51 472, 29 49 396 sowie EP-A-0 135 348 und 0 135 863).

Ebenso zählen unter diesen Oberbegriff insbesondere auch aromatische Verbindungen, die kernständiges Halogen, vorzugsweise Brom, aufweisen. Solche Verbindungen sind aus der DE-A-26 10 842 bekannt oder werden in der gleichzeitig eingereichten Patentanmeldung P 37 30 784.3 beschrieben.

Als Vertreter einer Kombination mit einem Thiazol sei eine mit 2-Benzoylmethylen-naphtho[1,2-d]thiazol genannt (DE-A-28 51 641 und DE-A-29 34 758). Ein Gemisch einer Trihalogenmethylverbindung mit N-Phenylacridon ist aus der DE-A-26 10 842 bekannt.

Ebenso stehen $\alpha$-Halogencarbonsäureamide (DE-A-27 18 200) oder Tribrommethyl-phenylsulfone (DE-A-35 03 113), die z.B. durch Benzophenon, Benzil oder Michlers Keton sensibilisiert werden können, zur Verfügung.

In der Regel können alle beschriebenen strahlungsempfindlichen Verbindungen durch Photosensibilisatoren in ihrer Effektivität unterstützt werden. Zu nennen sind z.B. Anthracen, Phenanthren, Pyren, 1,2-Benzanthren, Thiazine, Pyrazoline, Benzofurane, Benzophenone, Fluorenone, Anthrachinone sowie Cumarinderivate. Ihr Gehalt liegt bei 0,01 bis 5 Gew.-%, bezogen auf das Gewicht des strahlungsempfindlichen Gemisches.

Besonders bevorzugt sind vor allem Triazinderivate sowie aromatische Verbindungen mit kernständigem Halogen, insbesondere Brom, aus der DE-A-26 10 842 sowie der gleichzeitig eingereichten Patentanmeldung EP-A-0 318 649.

Von den in dieser Anmeldung genannten Verbindungen sind solche bevorzugt, die einen $pK_a$-Wert von 6 bis 10 aufweisen, ganz besonders diejenigen, die unter die allgemeine Formel II

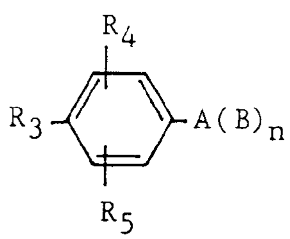

(II)

fallen, wobei

- $R_3$ Carboxyl, OR′ oder SR′,
- $R_4$, $R_5$ gleich oder verschieden sind und Wasserstoff, Chlor, Brom, Alkyl, ggf. substituiert durch Aryl-, Alkoxy-, Aryloxy-, Hydroxy- oder Fluoratome, Aryl, ggf. substituiert durch Alkoxy-, Aryloxy-, Hydroxy-oder Halogenatome,
- R′ Wasserstoff, Alkyl, ggf. substituiert durch Aryl-, Alkoxy-, Aryloxy-, Hydroxygruppen oder Fluoratome; Aryl, ggf. substituiert durch Alkoxy-, Aryloxy-, Hydroxygruppen oder Halogenatome; Acyl, Alkylsulfonyl, Arylsulfonyl, Alkoxycarbonyl, Triorganosilyl, Triorganostannyl oder eine Alkylen-, Arylen-, Bisacyl-, Sulfonyl-, Alkylendisulfonyl-, Arylendisulfonyl-, Diorganosilyl- oder Diorganostannylgruppe ist, deren zweite Valenz an das O-Atom einer weiteren Einheit der Formel I gebunden ist, wobei die Alkylen-

und Arylengruppen entsprechend substituiert sein können wie die Alkyl- und Arylreste,

und n 0 bis 3

bedeuten, wobei

für n = 0:

A Wasserstoff, Chlor, Brom, Alkyl, ggf. substituiert durch Alkoxy-, Aryloxy-, Hydroxy-, Arylreste oder Fluoratome, Aryl, ggf. substituiert durch Alkoxy-, Aryloxy-, Hydroxy-, Carboxylreste oder Halogenatome,

für n = 1:

A eine Einfachbindung, -0-, -S-, $-S0_2-$, -NH-,

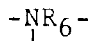,

Alkylen, Perfluoralkylen,

für n = 2:

A

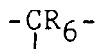 oder 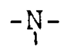

und

für n = 3:

A

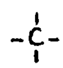

bedeutet,

sowie

B

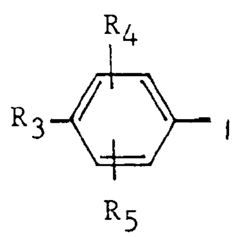

Carboxyl, substituiertes Carbonyl, insbesondere Alkyl- oder Arylcarbonyl, Carboxyalkyl sowie substituiertes Sulfonylimidocarbonyl und

$R_6$ Alkyl, insbesondere $(C_1-C_3)$Alkyl, oder Aryl, insbesondere Phenyl,

bedeuten.

Diese Verbindungen werden vorzugsweise erst bei energiereicher, d.h. kürzerwelliger Strahlung gespalten. Hierunter zählt neben der UV-Strahlung vor allem die Elektronen- oder die Röntgenstrahlung.

Neben monomeren strahlungsempfindlichen Säurebildnern können auch Polymere dieser Art eingesetzt werden. Bei Verwendung von monomeren Startern liegt deren Gehalt im Bereich von 0,01 bis 30 Gew.-%, insbesondere von 0,4 bis 20 Gew.-%, bezogen auf das Gewicht der strahlungsempfindlichen Mischung.

Werden polymere Starter verwendet, so kann ggf. auf ein polymeres Bindemittel verzichtet werden. Darüber hinaus kann der polymere Starter mit dem Bindemittel gemischt sein; es ist aber auch möglich, daß die monomeren Bestandteile eines nicht polymerisierten Starters mit monomeren Bestandteilen des Bindemittels zu einem Mischkondensat oder -polymerisat reagieren. Dies hat zur Folge, daß dessen Gehalt in Abhängigkeit von seinem Anteil am Gehalt des Polymeren in dieser Mischung auch über den beschriebenen Bereich bei monomeren Startern hinausgehen kann. Insbesondere werden Gehalte von größer als 50 Gew.-% und bis zu 100 Gew.-%, abzüglich des Gehaltes der Verbindung, die durch Säure spaltbar ist, in Anspruch genommen.

Das erfindungsgemäße strahlungsempfindliche Gemisch enthält ferner ggf. ein in Wasser unlösliches, in organischem Lösemittel und Alkali aber lösliches, zumindest quellbares Bindemittel. Hierunter zählen vor allem Phenolharze vom Novolak-Typ. Genannt werden Phenol-Formaldehyd-Harze, Kresol-Formaldehydharze, deren Mischkondensate und deren Mischungen.

Daneben können auch Vinylpolymerisate wie Polyvinylacetale, Polymethacrylate, Polyacrylate, Polyvinylether, Polyvinylpyrrolidone sowie Styrolpolymerisate, jeweils ggf. durch Comonomere modifiziert, selbst oder in Mi-schung mit anderen, eingesetzt werden.

Im besonderen sind zu nennen: Polymere von Styrol mit Einheiten von Alkenylsulfonyl-aminocarbonyloxy- oder Cycloalkenylsulfonylaminocarbonyloxy- (EP-A-0 184 804), Polymere der Acryl-, Methacryl-, Malein-, Itaconsäure etc. mit seitenständigen, vernetzenden -$CH_2$OR-Gruppen (EP-A-0 184 044), Polymerisate aus Vinylmonomeren mit Alkenylphenoleinheiten (EP-A-0 153 682), Polyvinylphenole als Novolakersatz (DE-C-2 322 230), polymere Bindemittel mit seitenständigen, phenolischen Hydroxylgruppen (EP-A-0 212 439 und 0 212 440), Styrol-Maleinsäureanhydrid-Mischpolymerisate (DE-A-3 130 987), Polymere aus ungesättigten (Thio)phosphinsäureiso(thio)cyanaten mit einem aktiven Wasserstoff enthaltenden Polymeren (deutsche Patentanmeldungen P 36 15 612.4 und P 36 15 613.2), Polymere mit Vinylacetat, Vinylalkohol und Vinylacetaleinheiten (EP-A-0 216 083) sowie Polyvinylacetale mit Einheiten aus Hydroxyaldehyden (deutsche Patentanmeldung P 36 44 162.7).

Die Menge des Bindemittels beträgt im allgemeinen 1 bis 90, insbesondere 5 bis 90 Gew.-%, vorzugsweise 50 bis 90 Gew.-%, bezogen auf das Gesamtgewicht der strahlungsempfindlichen Mischung.

Ferner können den erfindungsgemäßen strahlungsempfindlichen Gemischen ggf. Farbstoffe, Pigmente, Weichmacher, Netzmittel und Verlaufmittel, aber auch Polyglykole, Celluloseether, z.B. Ethylcellulose, zur Verbesserung spezieller Erfordernisse, wie Flexibilität, Haftung und Glanz, zugesetzt werden.

Vorzugsweise wird das erfindungsgemäße strahlungsempfindliche Gemisch in Lösungsmitteln wie Ethylenglykol, Glykolethern wie Glykolmonomethylether, Glykoldimethylether, Glykolmonoethylether oder Propylenglykolmonoalkylethern, insbesondere Propylenglykolmethylether; aliphatischen Estern wie Ethylacetat, Hydroxyethylacetat, Alkoxyethylacetat, γ-Butylacetat, Propylenglykolmonoalkyletheracetat, insbesondere Propylenglykolmethyletheracetat oder Amylacetat; Ethern wie Dioxan, Ketonen wie Methylethylketon, Methylisobutylketon, Cyclopentanon und Cyclohexanon; Dimethylformamid, Dimethylacetamid, Hexamethylphosphorsäureamid, N-Methyl-pyrrolidon, Butyrolacton, Tetrahydrofuran, und in Mischungen derselben gelöst. Besonders bevorzugt werden Glykolether, aliphatische Ester sowie Ketone.

Die mit den übrigen Bestandteilen des strahlungsempfindlichen Gemisches entstehenden Lösungen haben in der Regel einen Feststoffgehalt von 5 bis 60 Gew.-%, vorzugsweise bis 50 Gew.-%.

Erfindungsgemäß wird ferner ein strahlungsempfindliches Aufzeichnungsmaterial beansprucht, im wesentlichen bestehend aus einem Substrat und dem darauf aufgetragenen strahlungsempfindlichen Gemisch.

Als Substrate kommen alle Materialien in Frage, aus denen Kondensatoren, Halbleiter, mehrlagige gedruckte Schaltungen oder integrierte Schaltkreise bestehen bzw. hergestellt werden können. Insbesondere sind Oberflächen aus thermisch oxidiertem und/oder mit Aluminium beschichtetem Siliciummaterial zu nennen, die gegebenfalls auch dosiert sein können, einschließlich aller anderen in der Halbleitertechnologie üblichen Substrate wie beispielsweise Siliciumnitrid, Galliumarsenid, Indiumphosphid. Weiterhin kommen in Frage die aus der Flüssigkristalldisplay-Herstellung bekannten Substrate wie Glas, Indium-Zinnoxid; ferner Metallplatten und -folien, beispielsweise aus Aluminium, Kupfer, Zink; Bimetall- und Trimetallfolien, aber auch elektrisch nicht leitende Folien, die mit Metallen bedampft sind, ggf. mit Aluminium beschichtete $SiO_2$-Materialien und Papier. Diese Substrate können einer Temperatur-Vorbehandlung unterzogen werden, oberflächlich angerauht, angeätzt oder zur Erzielung erwünschter Eigenschaften, wie z. B. Erhöhung der Hydrophilie, mit Chemikalien behandelt sein.

In einer besonderen Ausführungsform kann das strahlungsempfindliche Gemisch zur besseren Haftung in dem Resist oder zwischen dem Resist und dem Substrat einen Haftvermittler enthalten. Bei Silicium- bzw. Siliciumdioxid-Substraten kommen hierfür Haftvermittler vom Aminosilan-Typ, wie z.B. 3-Aminopropyltriethoxysilan oder Hexamethyl-disilazan in Frage.

Beispiele für Träger, die zur Herstellung von photomechanischen Aufzeichnungsschichten wie Druckformen für den Hochdruck, den Flachdruck, den Siebdruck, den Tiefdruck sowie von Reliefkopien Verwendung finden können, sind Aluminiumplatten, ggf. anodisch oxidiert, gekörnte und/oder silikalierte Aluminiumplatten, Zinkplatten, Stahlplatten, die ggf. mit Chrom bearbeitet wurden, sowie Kunststoffolien oder Papier.

Das erfindungsgemäße Aufzeichnungsmaterial wird bildmäßig bestrahlt. Geeignete Quellen aktinischer Strahlung sind: Metallhalogenidlampen, Kohlebogenlampen, Xenonlampen und Quecksilberdampflampen. Bevorzugt wird eine Bestrahlung mit energiereicher Strahlung wie Laser, Elektronen- oder Röntgenstrahlung. Als Laser werden insbesondere Helium/Neon-Laser, Argon-Laser, Krypton-Laser sowie Helium/Cadmium-Laser genannt.

Die Beschichtungsmengen variieren in Abhängigkeit von ihrem Einsatzgebiet. Beim Aufbringen des strahlungsempfindlichen Gemisches z.B. auf eine Druckplatte wird die bevorzugte Beschichtungsmenge 0,5 bis 3,5 g/$m^2$ betragen. Im allgemeinen liegen die Schichtdicken zwischen 0,1 und 100 μm, insbesondere zwischen 1 und 10 μm.

Gegenstand der Erfindung ist ferner ein Verfahren zur Herstellung eines strahlungsempfindlichen Aufzeichnungsmaterials. Das Auftragen des strahlungsempfindlichen Gemisches auf das Substrat kann durch Aufsprühen, Fließbeschichten, Walzen, Schleuderbeschichten und Tauchbeschichten erfolgen. Danach wird das Lösungsmittel durch Verdampfen entfernt, so daß auf der Oberfläche des Substrates die strahlungsempfindliche Schicht zurückbleibt. Die Entfernung des Lösemittels kann gegebenfalls durch Erhitzen der Schicht auf Temperaturen von bis zu 150° C gefördert werden. Das Gemisch kann aber auch zunächst auf obengenannte Weise auf einen Zwischenträger aufgetragen werden, von dem aus es unter Druck und erhöhter Temperatur auf das endgültige Trägermaterial übertragen wird.

Anschließend wird die Schicht bildmäßig bestrahlt. Üblicherweise wird aktinische Strahlung verwendet, besonders bevorzugt sind UV-, Röntgen- oder Elektronenstrahlung. Üblicherweise werden zur Bestrahlung UV-Lampen verwendet, die Strahlung einer Wellenlänge von 200 bis 500 nm mit einer Intensität von 0,5 bis 60 $mW/cm^2$ aussenden. In der strahlungsempfindlichen Schicht wird anschließend durch Entwicklung ein Bildmuster freigelegt, in dem die Schicht mit einer Entwicklerlösung behandelt wird, die die bestrahlten Bereiche des Materials löst bzw. entfernt.

Als Entwickler werden Lösungen von alkalischen Reagenzien wie z.B. Silikaten, Metasilikaten, Hydroxiden, Hydrogen- bzw. Dihydrogenphosphaten, Carbonaten bzw. Hydrogencarbonaten, insbesondere von Alkali- oder Ammoniumionen, aber auch Ammoniak, organische Ammoniumhydroxide und dergleichen verwendet. Der Gehalt dieser Substanzen in der Entwicklerlösung beträgt im allgemeinen 0,1 bis 15 Gew.-%, vorzugsweise 0,5 bis 5 Gew.-%, bezogen auf das Gewicht der Entwicklerlösung. Besonders bevorzugt werden metallionenfreie Entwickler.

Zur Erhöhung der Resistenz gegen mechanische und chemische Einflüsse, insbesondere gegen Ätzmedien, können die entwickelten Schichten noch einige Zeit, z.B. 5 bis 40 Minuten, auf erhöhte Temperatur, z.B. oberhalb 100° C, erhitzt werden, wobei diese Wirkung noch durch Belichten mit UV-Strahlen unterstützt werden kann.

Die Herstellung der in den erfindungsgemäßen strahlungsempfindlichen Gemischen enthaltenen Acetale kann durch Kondensation des Aldehyds bzw. Ketons mit dem Alkohol unter azeotroper destillativer Entfernung des Reaktionswassers erfolgen. Bei einer anderen bekannten Variante wird der Aldehyd bzw. das Keton mit Orthoameisensäuretrimethylester zum Dimethylacetal umgesetzt. In einer zweiten Stufe wird dieses dann mit einem Alkohol zum gewünschten Acetal zur Reaktion gebracht. Überraschend wurde bei den im folgenden beschriebenen Herstellungsmethoden gefunden, daß bei der Herstellung der beanspruchten Acetale die gleichzeitige Zugabe von Orthoester und dem Alkohol zur Carbonylverbindung nicht zu den hier unerwünschten Umesterungsprodukten des Orthoesters führte, sondern nur die gewünschten Acetale entstanden.

Anhand der folgenden Beispiele soll die Herstellung einer im erfindungsgemäßen strahlungsempfindlichen Gemisch enthaltenen neuen säurespaltbaren Verbindung verdeutlicht werden.

Herstellungsbeispiel 1: Benzaldehyd-diphenoxyethylacetal

Zu einer Mischung aus 1 mol Benzaldehyd, 2 mol Phenoxyethanol und 1,1 mol Orthoameisensäuretrimethylester wurden unter Kühlung 0,5 g p-Toluolsulfonsäure gegeben. Es wurde 2 h bei Raumtemperatur gerührt. Anschließend wurde an die Mischungsapparatur ein Wasserstrahlvakuum angelegt und jeweils 2 h bei 20° C, 40° C und 60° C weitergerührt. Abschließend wurde die Reaktionsmischung bei 80° C und einem Druck von 0,1 Torr über einen Dünnschichtverdampfer gegeben. Gegebenenfalls kann eine weitere Destillation im Dünnschichtverdampfer erfolgen, bei entsprechend erhöhter Temperatur.

Das resultierende Acetal wurde mit $Na_2CO_3$, $K_2CO_3$ oder basischem Aluminiumoxid verrührt, wodurch der saure Katalysator neutralisiert wurde. Die festen Bestandteile wurden abgetrennt und das Filtrat im Vakuum eingeengt. Das Acetal konnte in der nun vorliegenden Form in einem Aufzeichnungsmaterial eingesetzt werden. Es wies einen Schmelzpunkt von 22-24° C auf; im IR-Spektrum war kein CO-Signal erkennbar; das NMR-Spektrum ($CDCl_3$) zeigte ein Acetalsignal bei $\delta$= 5,76 ppm.

In analoger Weise wurden die Acetale und Ketale der folgenden Herstellungsbeispiele dargestellt. Eine Zusammenstellung der Ausgangskomponenten (Carbonyl- und Alkoholkomponente) sowie der Daten des entstehenden Acetals bzw. Ketals (NMR-Signal und Siede- bzw. Schmelzpunkt) findet man in der folgenden Tabelle I:

Tabelle I

| Herstellungsbeispiele | Carbonylkomponente | Alkoholkomponente | NMR-Signal Acetal: δ [ppm] | Siedepunkt °C/Torr |
|---|---|---|---|---|
| H2 | Piperonal | 4-tert. Butylphenoxyethanol | 5,65 | |
| H3 | 2-Ethyl-butanal | Phenoxyethanol | 4,5 | 160/0,06 |
| H4 | Benzaldehyd | 4-(2-Hydroxyethoxy)-benzophenon | 5,49 | >170/0,1 |
| H5 | Benzaldehyd | 4-Bromphenoxyethanol | [illegible],76 | viskoses Öl |
| H6 | Benzaldehyd | Diethylenglykolmonophenylether | 5,65 | viskoses Öl |
| H7 | Benzaldehyd | Triethylenglykolmonophenylether | 5,60 | viskoses Öl |
| H8 | Cyclohexanon | Phenoxyethanol | | 129/0,01 Schmelzpunkt: 28 °C |
| H9 | Orthoameisensäure | Phenoxyethanol | 5,49 | >200/0,05 |

Zum Vergleich wurden die in Tabelle II zusammengestellten Acetale hergestellt, deren Alkoholkomponenten nicht unter die erfindungsgemäß beanspruchten fallen:

Tabelle II

| Herstellungsbeispiele | Carbonylkomponente | Alkoholkomponente | NMR-Signal Acetal: δ [ppm] | Siedepunkt °C/Torr |
|---|---|---|---|---|
| HV1 | Cyclohexanon | Polyglykol 200 | 1,25-1,75<br>3,45-3,75 | viskoses Öl |
| HV2 | Benzaldehyd | n-Hexanol | 5,50 | 128/0,1 |
| HV3 | Cyclohexanon | Methyldiglykol | 3,18<br>$CH_3O$-Gruppe | 140/0,01 |
| HV4 | Orthoameisensäure | n-Butyldiglykol | 5,33 | |

In Tabelle III sind die Siedepunkte (Torr) verschiedener im erfindungsgemäßen Gemisch beanspruchter Alkoholkomponenten der allgemeinen Formel $R-(O-CH_2CH_2)_n-OH$ zusammengestellt. Die Alkohole mit R = Methyl und n-Butyl sowie Hexanol sind als Vergleichsbeispiele diesen Verbindungen gegenübergestellt.

Tabelle III

| R | n = 1<br>°C/Torr | n = 2<br>°C/Torr | n = 3<br>°C/Torr |
|---|---|---|---|
| Methyl | 125/760 | 194/760 | |
| n-Butyl | 171/760 | 231/760 | 278/760 |
| Phenyl | 272/760 | 110/0,5 | 140/0,5 |
| 4-tert. Butylphenol | 155/ 15 | | |
| Benzophenon | F= 81°C | | |
| Hexanol | 156/760 | | |

Im folgenden sind die Anwendungsbeispiele zusammengestellt, die das erfindungsgemäße strahlungsempfindliche Gemisch enthalten, sowie entsprechende Vergleichsbeispiele. Die Mengen sind normalerweise in Gewichtsteilen (Gt) angegeben.

Beispiel 1

Es wurde eine Beschichtungslösung hergestellt aus

17 Gt eines Kresol-Formaldehyd-Novolaks mit einem Erweichungsbereich von 105-120° C,

5 Gt Benzaldehyd-diphenoxyethylacetal (Herstellungsbeispiel 1)
4 Gt Tetrabrom-Bisphenol A (Dow Chemical) in
74 Gt Propylenglykolmethyletheracetat.

Die Lösung wurde auf einen mit einem Haftvermittler (Hexamethyl-disilazan) behandelten Siliciumwafer bei 3.000 U/min aufgeschleudert. Nach der Trocknung bei 85° C für 30 min in einem Umluftofen wurde eine Schichtdicke von 1 µm erhalten. Bildmäßig bestrahlt wurde mit Synchrotron-Strahlung (BESSY, Berlin, Luftspalt 2 mm) einer Dosis von 32 mJ/cm² durch eine Gold-auf-Silicium-Maske. Den experimentellen Aufbau findet man bei A. Heuberger, "X-Ray Lithography", Microelectronic Engineering 3, 535-556 (1985). Nach einer Haltezeit von 5 min wurde mit einem alkalischen Entwickler folgender Zusammensetzung entwickelt:

5,3 Gt Natriummetasilikat x 9 $H_2O$,
3,4 Gt Trinatriumphosphat x 12 $H_2O$,
0,3 Gt Natriumdihydrogenphosphat und
91 Gt vollentsalztes Wasser.

Man erhielt ein fehlerfreies Bild mit allen Details der Maske, wobei auch Linien und Gräben von 0,3 µm fehlerfrei wiedergegeben wurden. Die Resistflanken zeigten Winkel von nahezu 90° (in einer Aufnahme eines Rasterelektronenmikroskops (REM)).

Beispiele 2 bis 9

Es wurde verfahren wie in Beispiel 1 mit dem Unterschied, daß anstelle des dort beschriebenen Acetals die Acetale aus den Herstellungsbeispielen H2 bis H9 der Tabelle I eingesetzt wurden. Dabei wurden in den Entwicklern aus Beispiel 1 und einem metallionenfreien Entwickler (wäßrige Lösung von Tetramethylammoniumhydroxid) die folgenden Resultate erzielt:

Tabelle IV

| Beispiel | Acetal | Entwicklungsdauer im Entwickler aus Beispiel 1 | Entwicklungsdauer im metallionen-freien Entwickler (0,3n) |
|---|---|---|---|
| 2 | H2 | 120 s (0,3n) | 10 s (>60 s) |
| 3 | H3 | 60 s (0,3n) | 10 s ( 60 s) |
| 4 | H4 | 150 s (0,3n) | 20 s (>60 s) |
| 5 | H5 | 25 s (0,6n) | 20 s ( 60 s) |
| 6 | H6 | 90 s (0,3n) | 10 s ( 50 s) |
| 7 | H7 | 50 s (0,6n) | 25 s (>60 s) |
| 8 | H8 | 90 s (0,6n) | 10 s (>60 s) |
| 9 | H9 | 80 s (0,3n) | 10 s ( 40 s) |

Alle untersuchten Acetale wiesen in beiden Entwicklertypen eine gute Entwickelbarkeit und auch dem aggressiveren metallionenfreien Entwickler gegenüber eine günstige Entwicklerresistenz der unbestrahlten Stellen auf. Die Wartezeit zwischen Bestrahlung und Entwicklung hatte keinen Einfluß auf das Auflösevermögen, sofern sie mindestens 10 min betrug und 8 h nicht überschritt.

Vergleichsbeispiele V1 bis V4:

Es wurde wie in Beispiel 1 verfahren, nur mit der Maßgabe, daß anstelle des dort eingesetzten Acetals die säurelabilen Verbindungen aus Tabelle II verwendet wurden. Die Ergebnisse sind in Tabelle V zusammengefaßt:

Tabelle V

| Vergleichsbeispiele | säurelabile Verbind. | Entwicklungsdauer im Entwickler aus Beispiel 1 (0,3n) | Entwicklungsdauer im metallionenfreien Entwickler (0,3n) | Bemerkungen |
|---|---|---|---|---|
| V1 | HV1 | 45 s | 6 s | gute Toleranz i.d. Wartezeit zwischen Bestrahlung und Entwicklung, aber geringe Entwicklerresistenz |
| V2 | HV2 | >120 s | 15 s | geringe Toleranz i.d. Wartezeit zwischen Bestrahlung und Entwicklung |
| V3 | HV3 | >120 s | 15 s | geringe Resistenz gegenüber dem metallionenfreien Entwickler |
| V4 | HV4 | 60 s | 15 s | geringe Resistenz gegenüber dem metallionenfreien Entwickler |

Um das thermische Verhalten des strahlungsempfindlichen Gemisches abschätzen zu können, wurden die Erweichungspunkte einiger erfindungsgemäßer Gemische miteinander verglichen. Die dabei erhaltenen Ergebnisse sind in Tabelle VI zusammengestellt:

Tabelle VI

| Gemisch enthaltend | Erweichungspunkt (°C) gemessen auf der "Kofler-Bank" |
|---|---|
| H5 | 123 |
| H6 | 120 |
| H9 | 106 |
| HV1 | 70 |
| HV2 | 110 |
| HV3 | 98 |
| HV4 | 100 |

Wie die vorliegenden Beispiele und Vergleichsbeispiele belegen, ist es nur bei Verwendung der erfindungsgemäß eingesetzten Alkoholkomponenten möglich, ein gutes Entwicklungsverhalten in metallionenhaltigen und metallionenfreien Entwicklern mit gutem Wärmestand und günstigem Plasmaätzverhalten zu erhalten. Die Verwendung anderer Aldehydkomponenten, entsprechend dem Stand der Technik, führen durchweg zu einem schlechten Eigenschaftsspektrum.

Beispiel 10

Es wurde eine Beschichtungslösung hergestellt, enthaltend
5 Gt eines Poly-(brenzkatechin-monomethacrylates),
0,1 Gt 2-(4-Ethoxy-naphth-1-yl)-4,6-bis-(trichlormethyl-s-triazin) und
1 Gt des Acetals aus Benzaldehyd mit 4-Brom-phenoxyethanol (H5).

Die Beschichtungslösung wurde in an sich bekannter Weise auf einen aus Aluminium bestehenden 0,3 mm dicken Druckplattenträger aufgetragen. Der Aluminiumträger war vorher elektrochemisch aufgerauht, anodisiert und mit Polyvinylphosphonsäure hydrophiliert worden. Nach dem Trocknen der Schicht wurde ein Schichtgewicht von 1,6 g/$m^2$ erhalten.

Die in dieser Weise hergestellte Offsetdruckplatte wurde mit einer Kopiervorlage bedeckt und mit Strahlung einer Quecksilberhochdrucklampe (5 kW) 20 s in einem Abstand von 110 cm bestrahlt. Mit einem Entwickler gemäß Beispiel 1 wurde entwickelt. Man erhielt eine positive Druckform mit hoher Auflösung, die in einer Offsetdruckmaschine 100.000 einwandfreie Drucke lieferte.

Vergleichsbeispiel V7

In diesem Vergleichsbeispiel wurde anstelle der Verbindung H5 ein Acetal aus Cyclohexanon und Polyglykol 200 (HV1) verwendet. Ansonsten entsprachen die Komponenten denen aus Beispiel 10. An einer Offsetdruckmaschine konnten allerdings weniger als 20.000 einwandfreie Drucke erzeugt werden.

**Patentansprüche**

**1.** Positiv arbeitendes strahlungsempfindliches Gemisch, das eine Verbindung enthält, die unter Einwirkung von aktinischer Strahlung eine Säure bildet, und eine Verbindung, die durch Säure spaltbar ist, dadurch gekennzeichnet, daß die durch Säure spaltbare Verbindung ein Acetal oder Ketal ist, das aus einem Aldehyd oder einem Keton und einem Alkohol der allgemeinen Formel I

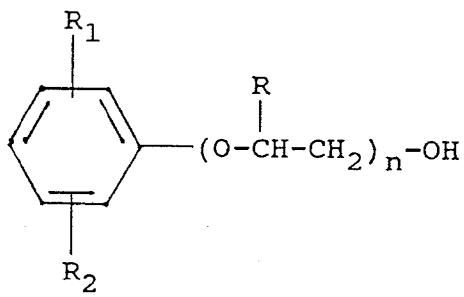

(I)

gebildet wird, in der

R Wasserstoff oder Alkyl darstellt,

$R_1$ und $R_2$ gleich oder verschieden sind und Wasserstoff, Hydroxy, Halogen, Cyano, Nitro, Alkyl, Alkoxy, Aryl, substituiertes Carbonyl, insbesondere Aryl- oder Alkylcarbonyl bedeuten, oder zusammen einen Ring formen, der mit $R_1$ und/oder $R_2$ substituiert sein kann, und

n 1 bis 3 ist.

2. Strahlungsempfindliches Gemisch nach Anspruch 1, dadurch gekennzeichnet, daß als Acetal ein Orthocarbonsäureester, ein Orthocarbonat oder ein Carbonsäureamidacetal eingesetzt wird.

3. Strahlungsempfindliches Gemisch nach Anspruch 1, dadurch gekennzeichnet, daß die Aldehyd- bzw. Ketonkomponente der durch Säure spaltbaren Verbindung einen Siedepunkt von oberhalb 155 °C und eine Löslichkeit im wäßrig-alkalischen Entwickler zwischen 0,1 und 100 g/l aufweist.

4. Strahlungsempfindliches Gemisch nach einem oder mehreren der Ansprüche 1 bis 3, dadurch gekennzeichnet, daß es ein in Wasser unlösliches, in wäßrigalkalischer Lösung aber lösliches Bindemittel enthält.

5. Positiv arbeitendes strahlungsempfindliches Aufzeichnungsmaterial, im wesentlichen bestehend aus einem Träger und einer strahlungsempfindlichen Schicht, dadurch gekennzeichnet, daß ein strahlungsempfindliches Gemisch gemäß den Ansprüchen 1 bis 4 verwendet wird.

6. Verfahren zur Herstellung des strahlungsempfindlichen Aufzeichnungsmaterials für mikroelektronische Schaltungen, dadurch gekennzeichnet, daß ein strahlungsempfindliches Gemisch gemäß den Ansprüchen 1 bis 4 auf ein geeignetes Substrat aufgebracht, dieses getrocknet, anschließend bildmäßig bestrahlt und schließlich mit einem metallionenhaltigen oder metallionenfreien, wäßrig-alkalischen Entwickler entwickelt wird.

## Claims

1. A positive-working radiation-sensitive mixture containing a compound which forms an acid under the action of actinic radiation, and an acid-cleavable compound, characterized in that the acid-cleavable compound is an acetal or ketal which is formed of an aldehyde or ketone and an alcohol of the general formula I

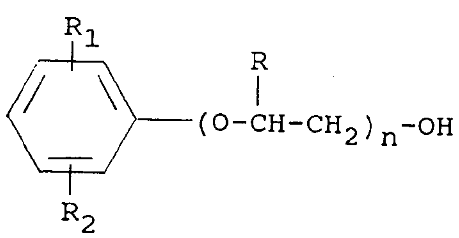

(I)

in which

R denotes hydrogen or alkyl,

$R_1$ and $R_2$ may be identical or different and denote hydrogen, hydroxyl, halogen, cyano, nitro, alkyl,

alkoxy, aryl, substituted carbonyl, in particular arylcarbonyl or alkylcarbonyl, or together form a ring which is optionally substituted by $R_1$ or $R_2$, and

n denotes 1 to 3.

2. A radiation-sensitive mixture as claimed in claim 1, characterized in that the acetal is an orthocarboxylate, orthocarbonate or a carboxamide acetal.

3. A radiation-sensitive mixture as claimed in claim 1, characterized in that the aldehyde or ketal component of the acid-cleavable compound has a boiling point above 155 °C and a solubility in an aqueous-alkaline developer of between 0.1 and 100 g/l.

4. A radiation-sensitive mixture as claimed in one or more of claims 1 to 3, characterized in that it contains a binder which is insoluble in water, but soluble in aqueous-alkaline solution.

5. A positive-working radiation-sensitive recording material, essentially comprising a support and a radiation-sensitive layer, characterized in that a radiation-sensitive mixture as claimed in any of claims 1 to 4 is used.

6. A process for the production of a radiation-sensitive recording material for microelectronic circuits, characterized in that a radiation-sensitive mixture as claimed in any of claims 1 to 4 is applied to a suitable substrate, and the latter is dried, subsequently irradiated imagewise and finally developed using a metal ion-containing or metal ion-free, aqueous-alkaline developer.

## Revendications

1. Composition sensible aux radiations, travaillant en positif, contenant un composé qui forme un acide sous l'effet d'un rayonnement actinique, et un composé qui est dissociable par un acide, caractérisée en ce que le composé dissociable par un acide est un cétal ou un acétal qui est formé à partir d'un aldéhyde ou d'une cétone et d'un alcool de formule générale I

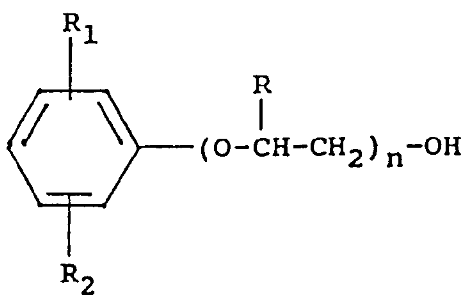

(I)

dans laquelle

R représente un atome d'hydrogène ou un groupe alkyle,

$R_1$ et $R_2$ sont identiques ou différents et représentent un atome d'hydrogène ou d'halogène ou un groupe hydroxy, cyano, nitro, alkyle, alcoxy, aryle ou un groupe carbonyle substitué, en particulier un groupe aryl- ou alkylcarbonyle, ou forment ensemble un cycle qui peut être substitué par $R_1$ et/ou $R_2$, et

n va de 1 à 3.

2. Composition sensible aux radiations selon la revendication 1, caractérisée en ce que, comme acétal, on utilise un ester d'acide orthocarboxylique, un orthocarbonate ou un acétal de carboxamide.

3. Composition sensible aux radiations selon la revendication 1, caractérisée en ce que le composant aldéhydique ou cétonique du composé dissociable par un acide présente un point d'ébullition supérieur à 155°C et une solubilité dans le révélateur aqueux-alcalin comprise entre 0,1 et 100 g/l.

4. Composition sensible aux radiations selon une ou plusieurs des revendications 1 à 3, caractérisée en ce qu'elle contient un liant insoluble dans l'eau mais soluble dans une solution aqueuse alcaline.

5. Matériau de reprographie sensible aux radiations, travaillant en positif, composé essentiellement d'un support et d'une couche sensible aux radiations, caractérisé en ce qu'est utilisée une composition sensible aux radiations selon les revendications 1 à 4.

6. Procédé pour la fabrication d'un matériau de reprographie sensible aux radiations, pour circuits microélectroniques, caractérisé en ce que l'on applique une composition sensible aux radiations selon les revendications 1 à 4 sur un substrat approprié, on sèche celui-ci, puis on l'insole selon l'image et enfin on la développe avec un révélateur aqueux-alcalin contenant des ions métalliques ou exempt d'ions métalliques.